# EUROPÄISCHE PATENTSCHRIFT

(11) **EP 1 971 271 B1**
(45) Veröffentlichungstag und Bekanntmachung des Hinweises auf die Patenterteilung: **13.03.2013**
(21) Anmeldenummer: 07702687.0
(22) Anmeldetag: 11.01.2007
(51) Int. Cl.: A61B 17/00, A61B 17/22, A61B 19/00

(54) **MEHRDRAHTINSTRUMENT, INSBESONDERE FÜR ENDOSKOPE**
MULTI-WIRE INSTRUMENT, IN PARTICULAR FOR ENDOSCOPES
INSTRUMENT A PLUSIEURS FILS, EN PARTICULIER POUR ENDOSCOPE

(30) Priorität: 11.01.2006 DE 102006002531
(43) Veröffentlichungstag der Anmeldung: 24.09.2008
(73) Patentinhaber: Epflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner
(86) Internationale Anmeldenummer: PCT/EP2007/000203
(87) Internationale Veröffentlichungsnummer: WO 2007/082671

(56) Entgegenhaltungen:
- DE-U1- 8 904 213
- DE-U1- 29 506 568
- FR-A1- 2 317 903
- US-A1- 2002 091 394
- US-B1- 6 187 017
- US-B1- 6 319 261

## Beschreibung

Die Erfindung bezieht sich auf ein Mehrdrahtinstrument nach dem Oberbegriff des Anspruchs 1.

Solche und ähnliche Mehrdrahtinstrumente werden insbesondere für Endoskope verwendet, beispielsweise in Anwendungen als Steinfangkorbinstrumente zum Einfangen von Gallen- oder Nierensteinen. Die Einzeldrähte bilden in ihrem Funktionszustand im distalen Funktionsabschnitt ein Funktionsdrahtgeflecht in Form eines Drahtkörbchens. Das Drahtkörbchen kann auch die Funktion einer Schlingen- oder Filtereinheit haben. Typischerweise kann der distale Funktionsabschnitt unter Zusammenfaltung des Funktionsdrahtgeflechts in ein Führungsrohr eingezogen und aus diesem unter Auffaltung des Funktionsdrahtgeflechts wieder herausbewegt werden. Dazu wird für die Einzeldrähte vorzugsweise ein superelastisches Material z.B. aus einer NiTi-Legierung, wie Nitinol, verwendet.

Bei einem gängigen Instrumententyp sind die Einzeldrähte proximal hinter ihrem distalen Funktionsabschnitt zusammengeführt und an einem proximal fortsetzenden, den Schaftabschnitt des Instruments bildenden Schaftdraht aus Rohr- oder Lizenmaterial fixiert, siehe z.B. die Patentschrift US 6.013.086. Im Gebrauch kann dieser Instrumententyp zu Schwierigkeiten führen, wenn die Verbindung zwischen den proximalen Enden der funktionsbildenden distalen Einzeldrähte und dem distalen Ende des Schaftdrahts z.B. unter Zugbelastung beim Einfangen eines Steins unbeabsichtigterweise reißt.

Als Abhilfe wird dazu in der Offenlegungsschrift DE 197 22 429 A1 vorgeschlagen, die Einzeldrähte des distalen Funktionsabschnitts durch bereichsweises axiales Schlitzen eines massiven oder rohrförmigen Rohdrahtes zu bilden, der im ungeschlitzten proximalen Bereich als schaftseitiger Zugstrang fungiert. Die axiale Schlitzung endet mit Abstand unter Belassung eines distalen Endstummels vor dem distalen Stirnende des Drahtrohlings, an dem ein z.B. kugel- oder halbkugelförmiges Abschlusselement angebracht sein kann.

In der nachveröffentlichten Offenlegungsschrift DE 10 2004 055 375 A1 ist ebenfalls eine einstückig aus einem Rohrstück gebildete Mehrdrahteinheit mit mehreren Drahtstücken beschrieben, in die das Rohrstück durch bereichsweise axiale Schlitzung aufgeteilt ist, wobei die Axialschlitze unter Belassung eines distalen Drahtverbindungsbereichs mit geeignet geringem axialem Abstand enden, so dass im Funktionszustand der Mehrdrahteinheit die Drahtstücke ein z.B. körbchenförmiges Funktionsdrahtgeflecht bilden und der distale Drahtverbindungsbereich zu einem im Wesentlichen spitzenlosen vorderen Endabschluss umgeformt ist.

In der nachveröffentlichten Offenlegungsschrift DE 10 2005 030 010 A1 ist eine Steinfangkorbeinheit beschrieben, bei welcher der Körbchenbereich durch Drahtstücke gebildet ist, die an ihrem distalen Ende zusammengeführt und an einem distalen Kopfstück festgelegt sind, das eine Sollbruchcharakteristik aufweist. Damit soll erreicht werden, dass das Kopfstück bei einem Überlastfall im Gebrauch die Drahtstücke an deren distalem Ende freigibt, so dass ein unerwünschtes, unkontrolliertes Reißen anderer Verbindungsstellen vermieden und/oder ein eingefangener Stein wieder freigegeben werden kann.

Die Gebrauchsmusterschrift DE 89 04 213 U1 offenbart ein Mehrdrahtinstrument der eingangs genannten Art in Form eines Lithotriptors mit einem Fangkörbchen, das von mehreren nach außen gebogen verlaufenden Fangsträngen aus Drahtmaterial gebildet wird, die an der Fangkörbchenspitze zusammen-laufen und mit einem Zugstrang aus mehreren Zugdrähten verbunden sind, wobei der Zugstrang aus einem zweifach verseilten Drahtseil besteht, dessen Litzenanzahl mindestens der Anzahl der Fangstränge des Fangkörbchens entspricht, die von verformten Litzen des Drahtseils gebildet werden, die vom Drahtseil aus bis zur Fangkörbchenspitze durchlaufen und dort mit ihren freien Enden aneinander befestigt sind. An einem proximalen Fußbereich des Fangkörbchens werden die Litzen des Drahtseils von einer Hülse zusammengehalten, in der außerdem die ggf. übrigen, nur im Schaftabschnitt verlaufenden Litzen distal enden. In einem proximal an den Fußbereich des Fangkörbchens anschließenden Teil des Schaftabschnitts sind die Litzen voneinander entseilt und verlaufen unverdrillt, wodurch die Zugstrangflexibilität erhöht werden soll. In gleicher Weise laufen die fangkorbbildenden Litzen in ihrem Fangkorbabschnitt und in ihrem daran anschließenden distalen Endbereich unverdrillt, wobei sie mit ihren freien distalen Enden durch Löten oder Klemmen unter Bildung einer Art Sollbruchstelle aneinander fixiert sind.

In der Patentschrift US 6.187.017 B1 ist ein Steinfangkorbinstrument mit einer spezifischen Fangkorbgestaltung offenbart, bei der sich je zwei paarbildende Einzeldrähte anschließend an den korbbildenden Abschnitt in distaler Richtung zunächst unter Bildung eines ersten Kreuzungspunktes überkreuzen, sich dann radial nochmals voneinander entfernen und anschließend wieder unter Bildung eines zweiten Kreuzungspunktes sich überkreuzend zusammengeführt sind, um dann in rein axialer Richtung auf Stoß an einem atraumatischen zylindrischen Abschlusskörper fixiert zu werden, wobei an den beiden Kreuzungspunkten der eine Draht beides Mal unter oder über dem anderen Draht oder einmal über und das andere Mal unter dem anderen Draht liegen kann. Mit ihrem proximalen Ende sind die Einzeldrähte direkt nach ihrem korbbildenden Abschnitt an einem separaten Schaftkörper fixiert.

Speziell für medizinische Anwendungen z.B. zur Nierensteinentfernung ist ein möglichst geringer Querschnitt bzw. Durchmesser für die Mehrdrahtinstrumente gewünscht, um diese problemlos in entsprechende Gewebekanäle einführen zu können. Gleichzeitig muss das von den Einzeldrähten bereitgestellte Funktionsdrahtgeflecht die für seine Funktion erforderlichen Eigenschaften insbesondere hinsichtlich Stabilität bzw. Festigkeit und Flexibilität bzw. Steifigkeit gewährleisten.

Der Erfindung liegt als technisches Problem die Bereitstellung eines Mehrdrahtinstrumentes der eingangs genannten Art zugrunde, das sich mit relativ geringem Aufwand und bei Bedarf mit vergleichsweise geringem Querschnitt bzw. Durchmesser auch in seinem Schaftabschnitt realisieren lässt und bei dem die Gefahr minimiert ist, dass Verbindungsstellen in unerwünschter Weise im Gebrauch reißen.

Die Erfindung löst dieses Problem durch die Bereitstellung eines Mehrdrahtinstruments mit den Merkmalen des Anspruchs 1. Bei diesem Mehrdrahtinstrument sind die Einzeldrähte am distalen Ende durch eine jeweilige Sollbruchverbindung direkt aneinander oder an einem distalen Abschlusskörper festgelegt und erstrecken sich einstückig vom distalen Ende zum proximalen Endbereich des Mehrdrahtinstruments, wobei der Schaftabschnitt von den dort zusammengeführten und aneinander gehaltenen Einzeldrähten gebildet ist.

Da die Einzeldrähte vom distalen Ende bis zum proximalen Endbereich des Mehrdrahtinstruments einstückig durchgeführt sind, gibt es keinen weiteren Schaftdraht, an dem die Einzeldrähte zu fixieren sind und folglich keine Verbindungsstelle der proximalen Enden der Einzeldrähte mit dem distalen Ende eines proximal anschließenden Schaftabschnitts. Vielmehr bilden die Einzeldrähte im entsprechenden Bereich selbst den gesamten Schaftabschnitt. Damit entfällt die Gefahr, dass derartige Verbindungsstellen im Gebrauch unter Belastung unerwünschterweise reißen. Vielmehr wird dadurch, dass die Sollbruchverbindungen am distalen Ende der Einzeldrähte vorgesehen sind, dafür gesorgt, dass sich an diesen Sollbruchstellen die Einzeldrähte definiert an ihrem distalen Ende von der Verbindungsstelle lösen können, so dass sich das von den Einzeldrähten gebildete Funktionsdrahtgeflecht an seinem distalen Ende öffnen kann. Dies ermöglicht z.B. in Anwendung als Steinfangkorbinstrument ein distales Freigeben eines bereits eingefangenen Steins im Überlastfall, wodurch vermieden wird, dass das Instrument an anderer Stelle reißt und eventuell Teile des Instruments im Gewebe verbleiben und/oder ein zu großer, eingefangener Stein nicht mehr freigegeben werden kann. Da der Schaftabschnitt von den zusammengeführten Einzeldrähten selbst gebildet ist, lässt er sich mit vergleichsweise geringem Querschnitt realisieren, der im Wesentlichen der Summe der Einzeldrahtquerschnitte entspricht.

Gemäß der Erfindung sind die Einzeldrähte im gesamten Schaftabschnitt und somit distal bis zum Übergang in den körbchenbildenden Funktionsabschnitt miteinander verdrillt. Dies ermöglicht die Bereitstellung eines hoch belastbaren Schaftabschnitts bei vergleichsweise geringem Querschnitt auch ohne Einsatz weiterer Hilfsmittel wie Führungs- oder Fixierhülsen oder dgl.

Gemäß der Erfindung sind veitehil die Einzeldrähte in einem proximal an die Sollbruchverbindungen anschließenden distalen Endbereich miteinander so verdrillt sind, dass sie entlang dieser Verdrillung aneinander anliegen. Diese Maßnahme ist zusätzlich zur Maßnahme der distal bis direkt zum körbchenbildenden Funktionsabschnitt durchgehenden Verdrillung im Schaftabschnitt vorgesehen und stellt eine erhöhte Belastbarkeit der Sollbruchverbindung der distalen Einzeldrahtenden bereit.

In vorteilhafter Weiterbildung der Erfindung nach Anspruch 2 sind die Einzeldrähte im körbchenbildenden Funktionsabschnitt unverdrillt, so dass sie dort ein entsprechendes Drahtkörbchen mit sich unverdrillt in zugehörigen Längsebenen erstreckenden Korbdrahtabschnitten bilden.

In einer Ausgestaltung der Erfindung nach Anspruch 3 beinhaltet der distale Abschlusskörper ein halbkugel- oder vollkugelförmiges Abschlusselement, wodurch dem distalen Ende des Instruments gewünschte atraumatische Eigenschaften verliehen werden.

In einer Ausgestaltung der Erfindung nach Anspruch 4 weist der distale Abschlusskörper ein Abschlusselement zum Fixieren der distalen Einzeldrahtenden sowie eine proximal an das Abschlusselement anschließende Schutzhülse auf, in der die Einzeldrähte mit ihrem distalen Endbereich gefasst sind. Die Schutzhülse kann dazu dienen, der jeweiligen Sollbruchverbindung der distalen Einzeldrahtenden eine gewünschte Festigkeit z.B. gegenüber einem Aufbiegen der Einzeldrähte in einen körbchenbildenden Funktionszustand zu verleihen.

In einer Ausgestaltung der Erfindung nach Anspruch 5 ist die distal endseitige Verdrillung der Einzeldrähte gleichartig zu derjenigen im Schaftabschnitt, d.h. sie weist im Wesentlichen die gleichen Verdrillungsparameter auf, insbesondere eine im Wesentlichen gleich große Ganghöhe der schraubenförmigen Verdrillungswindungen.

In einer Ausgestaltung der Erfindung nach Anspruch 6 sind die Einzeldrähte mit ihrem proximalen Ende direkt aneinander oder an einem proximalen Abschlusskörper fixiert und/oder im proximalen Endbereich in einem Griffrohrelement aufgenommen. Dies realisiert vorteilhafte Gestaltungen des Mehrdrahtinstruments in seinem proximalen Endabschnitt.

Für die Einzeldrähte ist, wie im Anspruch 7 angegeben, z.B. monofiles Drahtmaterial oder Litzendrahtmaterial verwendbar.

Vorteilhafte Ausführungsformen der Erfindung sind in den Zeichnungen dargestellt und werden nachfolgend beschrieben. Nur Ausführusformen mit im distalen Endbereil verdrillten Einzeldrählen sind Teil der Erfindung. Hierbei zeigen:
- Fig. 1: eine schematische Seitenansicht eines endoskopischen Steinfanginstruments mit abgenommenem Handgriff,
- Fig. 2: eine detailliertere Seitenansicht eines vorderen Teils des Steinfangkorbinstruments von Fig. 1,
- Fig. 3: eine Seitenansicht eines für das Steinfangkorbinstrument von Fig. 1 verwendbaren Mehrdrahtinstruments,
- Fig. 4: eine detailliertere Seitenansicht eines hinteren Endbereichs des Mehrdrahtinstruments von Fig. 3,
- Fig. 5: eine Seitenansicht eines hinteren Endbereichs eines modifizierten Mehrdrahtinstruments,
- Fig. 6: eine detailliertere Seitenansicht eines Bereichs Y des Mehrdrahtinstruments von Fig. 3,
- Fig. 7: eine detailliertere Seitenansicht eines vorderen Endbereichs X des Mehrdrahtinstruments von Fig. 3 mit halbkugelförmigem Abschlusselement und Schutzhülse,
- Fig. 8: eine Ansicht entsprechend Fig. 7 für eine Variante mit der erfindungsgemäβen, zusätzlicher Verdrillung der Einzeldrähte im distalen Endbereich,
- Fig. 9: eine Ansicht entsprechend Fig. 7 für eine Variante ohne Schutzhülse und mit kugelförmigem Abschlusselement,
- Fig. 10: die Ansicht von Fig. 9 mit gebrochener Sollbruchverbindung eines der Einzeldrähte,
- Fig. 11: eine Ansicht entsprechend Fig. 9 für eine Variante mit der erfindungsgemäβen, zusätzlicher Verdrillung der Einzeldrähte im distalen Endbereich,
- Fig. 12: eine Ansicht entsprechend Fig. 9 für eine Variante mit halbkugelförmigem Abschlusselement,
- Fig. 13: eine Seitenansicht einer Variante des Mehrdrahtinstruments von Fig. 3 mit proximalem Endbereich entsprechend Fig. 5 und distalem Endbereich entsprechend Fig. 9,
- Fig. 14: eine Seitenansicht eines Rohzustands des Mehrdrahtinstruments von Fig. 13 in einem anfänglichen Fertigungsstadium,
- Fig. 15: eine Seitenansicht entsprechend Fig. 13 für eine Variante mit vier statt zwei Einzeldrähten und
- Fig. 16: eine Seitenansicht entsprechend Fig. 13 für eine Variante mit sechs statt vier Einzeldrähten.

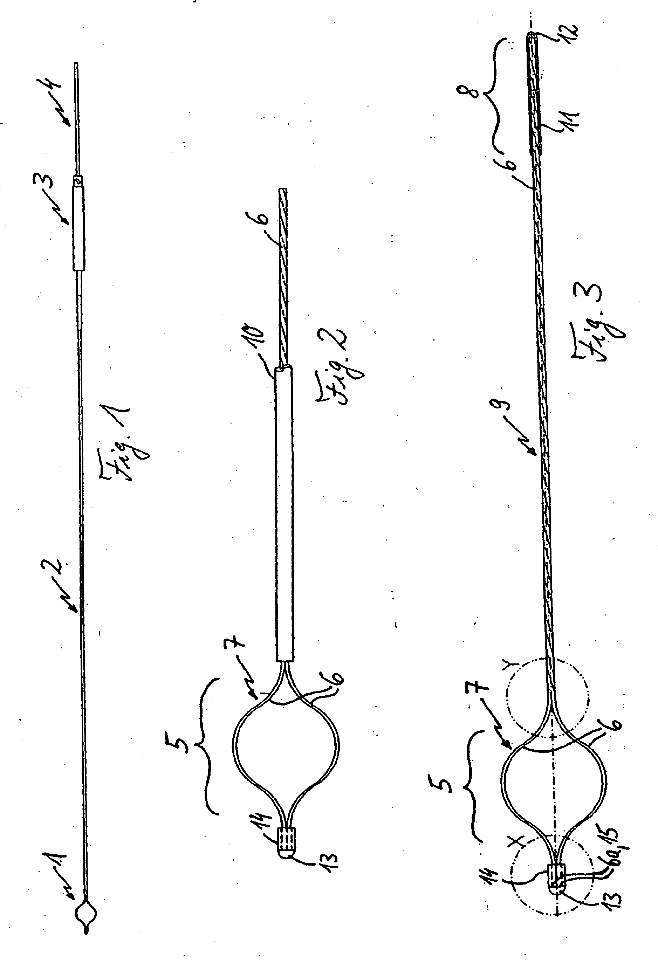

Nachfolgend werden vorteilhafte Ausführungsbeispiele unter Bezugnahme auf die Zeichnungen näher erläutert, in denen zum leichteren Verständnis identische oder funktionell äquivalente Elemente mit gleichen Bezugszeichen markiert sind. Das in Fig. 1 gezeigte Steinfangkorbinstrument weist einen an sich üblichen Aufbau mit einem distalen Funktionsbereich 1 und einem daran proximal anschließenden Schaftbereich 2 auf, der an seinem hinteren Ende ein Griffanschlussrohr 3 durchquert und einen sogenannten Zugrohrabschnitt 4 bildet. Kernbestandteil eines solchen Steinfangkorbinstruments, wie es z.B. zur endoskopischen Gallen- oder Nierensteinentfernung eingesetzt wird, ist ein Mehrdrahtinstrument, wie es in einer erfindungsgemäßen Realisierung in Fig. 3 gezeigt ist.

Wie aus Fig. 3 ersichtlich, beinhaltet das dort gezeigte Mehrdrahtinstrument einen distalen Funktionsabschnitt 5, in welchem mehrere Einzeldrähte 6, im gezeigten Beispiel zwei Einzeldrähte, im gezeigten Funktionszustand ein körbchenbildendes Funktionsdrahtgeflecht in Form einer Körbchen- oder Schlingeneinheit 7 bilden, die zum Einfangen von Steinen geeignet ist. Die Einzeldrähte 6 sind an ihrem distalen Ende zusammengeführt und miteinander verbunden. Proximal sind die Einzeldrähte 6 im Anschluss an den Funktionsabschnitt 5 miteinander verdrillt und erstrecken sich in dieser Weise einstückig bis zu einem proximalen Endbereich 8 des Mehrdrahtinstruments. Dabei bilden sie mit ihrem verdrillten Bereich den gesamten betreffenden Schaftabschnitt 9 des Mehrdrahtinstruments vom proximalen Ende bis direkt zum Übergang in den körbchenbildenden Funktionsabschnitt 5.

In an sich bekannter Weise ist das Mehrdrahtinstrument bei Einsatz im Steinfangkorbinstrument gemäß Fig. 1 mit seinem Schaftabschnitt 9 axial beweglich in einem flexiblen Führungsrohr bzw. Führungsschlauch 10 geführt, wie in Fig. 2 für einen vorderen Teil des Steinfangkorbinstruments von Fig. 1 detaillierter gezeigt. An das hintere Ende des Führungsrohrs 10 ist, wie aus Fig. 1 ersichtlich, das Griffanschlussrohr 3 angekoppelt, an das seinerseits in üblicher Weise ein nicht gezeigter Handgriff zur Betätigung des Steinfangkorbinstruments anschließbar ist. In Fig. 2 befindet sich das Mehrdrahtinstrument in seinem aus dem Führungsrohr 10 nach vorne herausbewegten Zustand, in welchem der distale Funktionsabschnitt 5 vom Führungsrohr 10 freigegeben ist und folglich die Einzeldrähte 6 dort ihren körbchen- bzw. schlingenbildenden Funktionszustand 7 einnehmen. Von diesem Zustand kann das Mehrdrahtinstrument axial in einen eingezogenen Zustand bewegt werden, in welchem das Führungsrohr 10 mit seinem distalen Bereich den distalen Funktionsabschnitt 5 des Mehrdrahtinstruments aufnimmt, wobei sich die Einzeldrähte 6 entsprechend zusammenfalten. Von diesem eingezogenen, zusammengefalteten Zustand kann das Mehrdrahtinstrument dann wieder unter körbchen- bzw. schlingenbildender Auffaltung der Einzeldrähte 6 im distalen Funktionsabschnitt 5 wieder nach vorne herausbewegt werden.

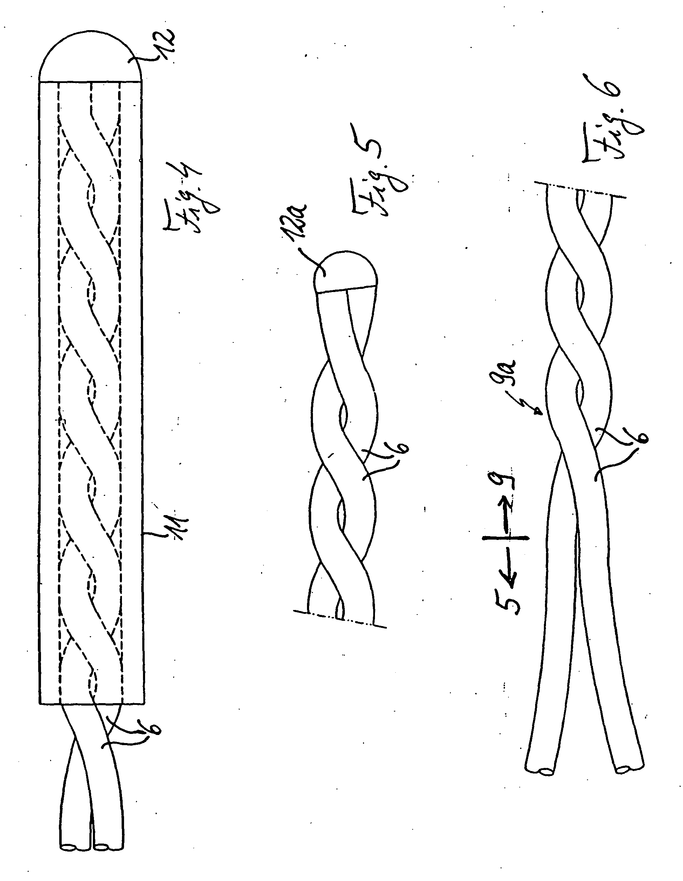

Im proximalen Endbereich 8 ist der aus den verdrillten Einzeldrähten 6 gebildete Schaftabschnitt 9 des Mehrdrahtinstruments von Fig. 3 in einer Griffhülse 11 aufgenommen und stirnseitig durch ein halbkugelförmiges Abschlusselement 12 bündig abgeschlossen, wie in der Detailansicht von Fig. 4 deutlicher zu erkennen. In einer in Fig. 5 gezeigten Variante sind die verdrillten Einzeldrähte 6 an ihrem proximalen Ende ohne aufnehmende Griffhülse mit einem halbkugelförmigen Abschlusselement 12a abgeschlossen.

Wie aus den vorstehenden Erläuterungen ersichtlich, besteht somit das fertige Steinfanginstrument von Fig. 1 aus dem Mehrdrahtinstrument von Fig. 3 sowie dem Führungsrohr 10 nebst angekoppeltem Griffanschlussrohr 3 und dem daran angekoppelten, nicht gezeigten Handgriff, wobei sich der Schaftabschnitt 9 des Mehrdrahtinstruments durch das Führungsrohr 10 und das Griffanschlussrohr 3 hindurch erstreckt und mit seinem proximalen Endbereich 8 den Zugrohrabschnitt 4 des Steinfanginstruments bildet.

Fig. 6 zeigt in einer Detailansicht den Übergang der Einzeldrähte 6 von ihrem körbchen- bzw. schlingenbildend aufgebogenen, unverdrillten Funktionszustand im distalen Funktionsabschnitt 5 direkt zum proximal anschließenden, verdrillten distalen Endbereich 9a des Schaftabschnitts 9.

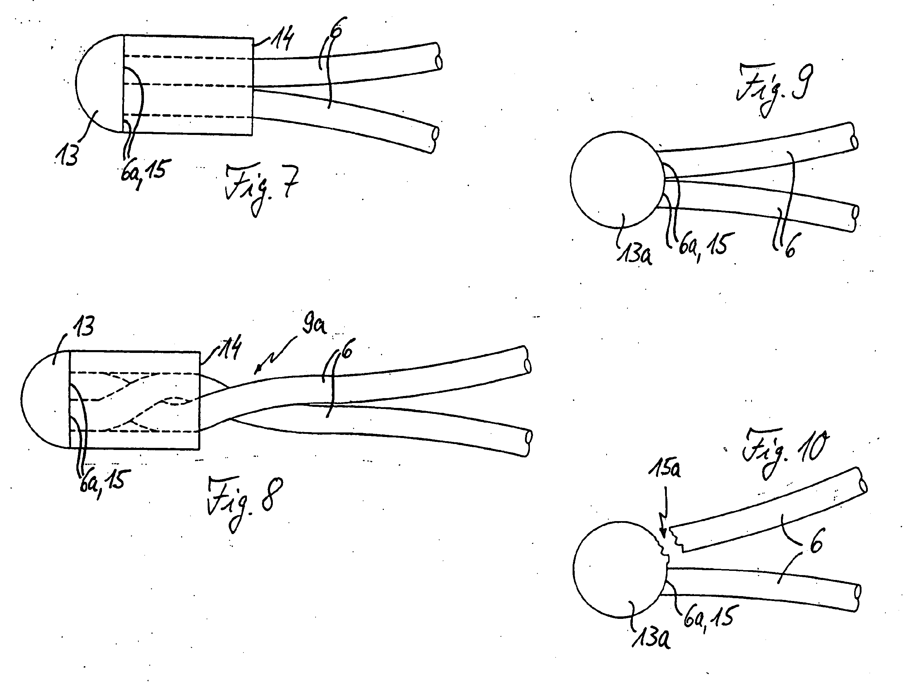

Fig. 7 zeigt detaillierter den distalen Endbereich X des Mehrdrahtinstruments von Fig. 3. Wie daraus ersichtlich, sind die Einzeldrähte 6 mit ihren distalen Enden 6a auf Stoß an einem halbkugelförmigen distalen Abschlusselement 13 fixiert, wobei diese Verbindungen jeweils als Sollbruchverbindung 15 ausgelegt sind, d.h. die Festigkeit dieser Verbindungen ist auf einen vorgebbaren Maximalwert eingestellt. Wenn die Belastung der Sollbruchverbindungen 15 der distalen Einzeldrahtenden 6a mit dem distalen Abschlusselement 13 im Gebrauch diesen Maximalwert überschreitet, reißt erwünschtermaßen diese Verbindung 15, so dass sich einer oder mehrere der Einzeldrähte 6 mit ihrem distalen Ende lösen und sich das von den Einzeldrähten 6 gebildete Funktionsdrahtgeflecht 7 distal öffnet. Ein eingefangener Stein kann auf die Weise wieder problemlos freigegeben werden, wenn sich zeigt, dass er nicht unter normaler Belastung durch das Steinfanginstrument herausbewegt oder zerkleinert werden kann.

An dem distalen Abschlusselement 13 ist des Weiteren eine die Einzeldrähte 6 in ihrem distalen Endbereich umgebende Schutzhülse 14 außenbündig fixiert. Mit dieser Schutzhülse 14 kann die Sollbruchverbindung der distalen Einzeldrahtenden 6a am distalen Abschlusselement 13 vor allem gegenüber Belastungen geschützt werden, die durch ein radiales Auseinanderbiegen der Einzeldrähte 6 in ihrem distalen Funktionsabschnitt resultieren, so dass ein unerwünschtes vorzeitiges Brechen dieser Sollbruchverbindungen 15 aufgrund solcher Belastungseinwirkungen vermieden wird.

Fig. 8 zeigt eine sehr vorteilhafte Variante des Beispiels von Fig. 7 hinsichtlich der distalen Endbereichgestaltung. Während im Beispiel von Fig. 7 die Einzeldrähte 6 im distalen Endbereich ohne Verdrillung zusammengeführt und in der Schutzhülse 14 bis zum distalen Abschlusselement 13 geführt sind, sind im Beispiel von Fig. 8 die Einzeldrähte 6 von ihrem distalen Funktionsabschnitt 5 aus nach vorn unter Verdrillung zusammengeführt und in der Schutzhülse 14 bis zum distalen Abschlusselement 13 geführt. Die distale Verdrillung der Einzeldrähte 6 bedeutet im vorliegend benutzten, üblichen Sinn dieses Begriffs, dass die Einzeldrähte entlang dieses Verdrillungsbereichs einander umschlingend durchgehend aneinander anliegen. Dies bietet einen zusätzlichen Belastungsschutz für die Sollbruchverbindung 15 der distalen Einzeldrahtenden 6a am distalen Abschlusselement 13. Vorzugsweise ist die distale Verdrillung der Einzeldrähte 6 gleichartig zur proximalen Verdrillung der Einzeldrähte 6 im Schaftabschnitt 9 realisiert. Dabei soll die Charakterisierung "gleichartig" bedeuten, dass die eine oder mehreren schraubenförmigen Windungen mit im Wesentlichen gleichen Windungsparametern für die beiden Verdrillungen realisiert sind, insbesondere was die Ganghöhe und den Außendurchmesser der Windungen betrifft. Dabei kann die distale Verdrillung gleichsinnig oder alternativ gegensinnig zur Verdrillung im Schaftabschnitt 9 realisiert sein.

Fig. 9 zeigt eine weitere Variante für die Gestaltung des distalen Endbereichs X des Mehrdrahtinstruments von Fig. 3 oder eines beliebigen anderen Mehrdrahtinstruments. Bei dieser Variante, die nicht Teil der beansprudlen Erfindung ist, sind die Einzeldrähte 6 unverdrillt und ohne distale Schutzhülse bis zu einem kugelförmigen distalen Abschlusselement 13a geführt und an diesem mit ihren distalen Enden 6a unter Bildung der jeweiligen Sollbruchverbindung 15 fixiert.

Fig. 10 zeigt die Ansicht von Fig. 9 für eine Situation, in der sich einer der Einzeldrähte 6 an seiner Sollbruchverbindung 15 vom distalen Abschlusselement 13a aufgrund einer Überlastung in gewünschter Weise gelöst hat. Durch die gerissene Verbindung 15a kann sich der gelöste Einzeldraht und damit die von den in diesem Beispiel zwei Einzeldrähten gebildete Körbchen-/Schlingeneinheit insgesamt distal öffnen, um dadurch z.B. einen bereits eingefangenen Stein wieder freizugeben. Ein solcher Überlastfall kann beispielsweise bewusst herbeigeführt werden, wenn sich herausstellt, dass ein eingefangener Stein nicht herausbewegt oder zerkleinert werden kann und daher vom Steinfangkorbinstrument wieder freigegeben werden soll, um es ohne Stein wieder aus einem Gewebekanal herausziehen zu können.

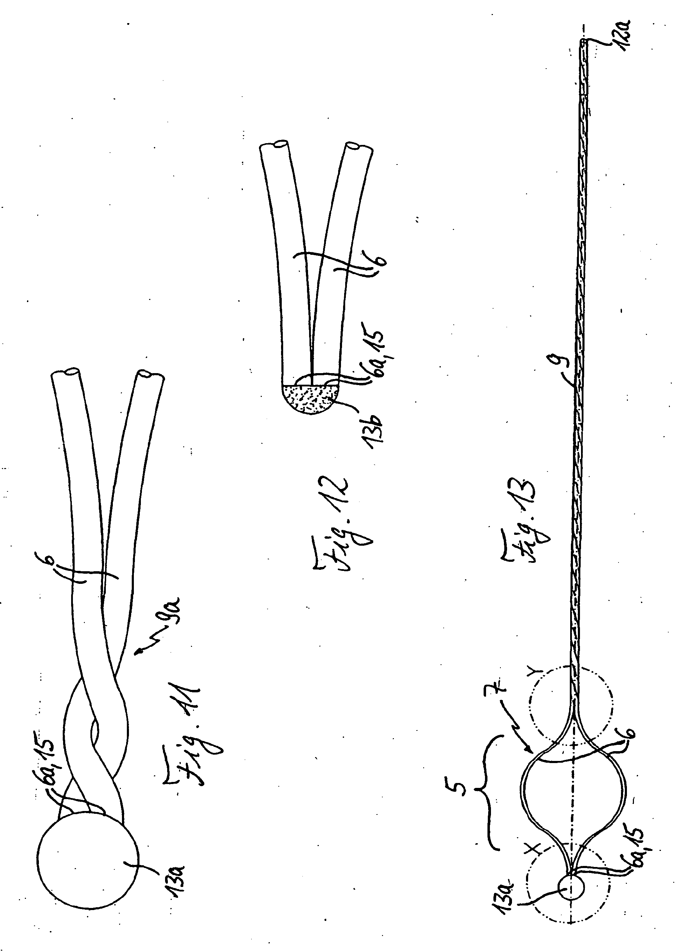

Fig. 11 zeigt eine erfindungsgemäβe variante von Fig. 9, bei der die vom distalen Funktionsabschnitt aus zusammengeführten Einzeldrähte 6 in ihrem distalen Endbereich bis zur Fixierung ihrer distalen Enden 6a durch die Sollbruchverbindungen 15 am kugelförmigen Abschlusselement 13a verdrillt sind. Dies erhöht wie im Beispiel von Fig. 8 die Festigkeit der Sollbruchverbindungen 15 insbesondere gegen ein Aufbiegen der Einzeldrähte 6 in ihrem körbchen-/schlingenbildenden distalen Funktionsabschnitt.

Fig. 12 zeigt eine weitere Variante für die Gestaltung des distalen Endbereichs des erfindungsgemäßen Mehrdrahtinstruments wiederum für den Fall von zwei Einzeldrähten 6 entsprechend dem Mehrdrahtinstrument von Fig. 3. Bei dieser Variante sind die Einzeldrähte 6 von ihrem distalen Funktionsabschnitt aus nach vorne zur bündigen Fixierung ihrer distalen Enden 6a durch die jeweilige Sollbruchverbindung 15 an einem halbkugelförmigen Abschlusselement 13b zusammengeführt.

Fig. 13 zeigt in einer Ansicht entsprechend Fig. 3 eine nicht leanspruchte Variante des Mehrdrahtinstruments, bei der die im Schaftbereich 9 verdrillten Einzeldrähte in ihrem proximalen Endbereich entsprechend Fig. 5 durch das proximale Abschlusselement 12a abgeschlossen sind und in ihrem distalen Endbereich anschließend an den distalen Funktionsabschnitt 5 entsprechend Fig. 9 unverdrillt zum kugelförmigen distalen Abschlusselement 13a geführt und an diesem durch die Sollbruchverbindungen 15 mit ihren distalen Enden 6a fixiert sind. Erfindungsgemäβ enden die Einzeldrähte distal ebenfalls mit einer Verdrillung analog zum Ausführungsbeispiel von Fig. 11.

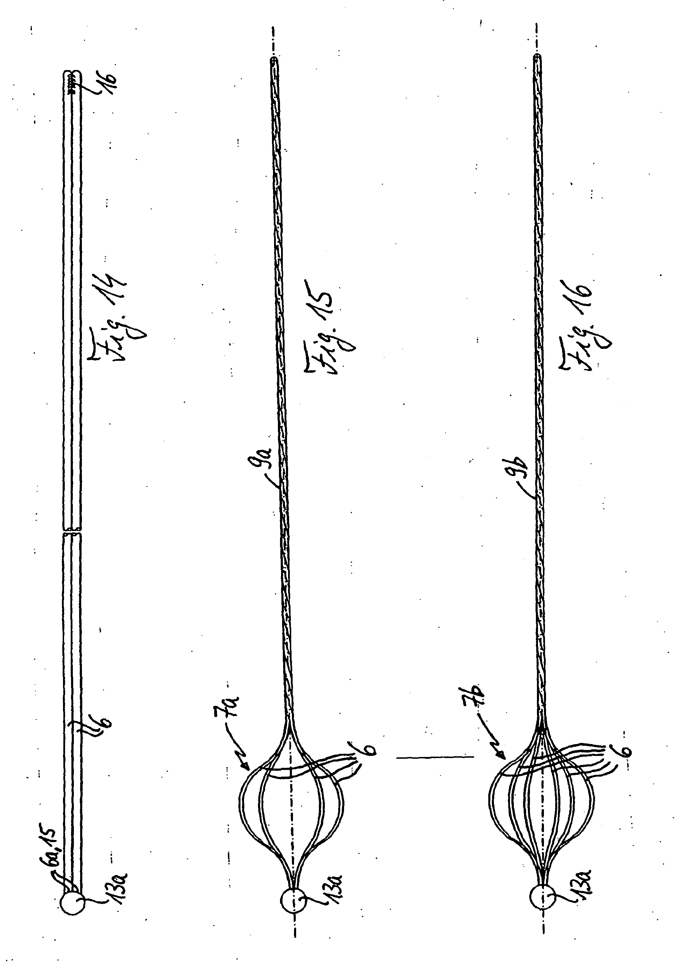

Das Mehrdrahtinstrument ist relativ einfach herstellbar, wie nachstehend am Beispiel des Mehrdrahtinstruments von Fig. 13 veranschaulicht. Fig. 14 zeigt das Mehrdrahtinstrument von Fig. 13 in einem anfänglichen Fertigungsstadium. Wie daraus ersichtlich, werden zunächst die Einzeldrähte 6 an ihrem proximalen Ende 6a durch die Sollbruchverbindungen 15 am kugelförmigen distalen Abschlusselement 13a fixiert und in ihrem proximalen Endbereich wie gezeigt durch eine Schweißverbindung 16 aneinander und/oder mit ihren proximalen Stirnenden an einem proximalen Abschlusselement angebracht. Anschließend werden dann die Einzeldrähte im distalen Funktionsabschnitt in ihre gewünschte Konfiguration gebogen, um das geforderte Funktionsdrahtgeflecht zu bilden, und im proximal anschließenden Schaftabschnitt in gewünschter Weise verdrillt.

Fig. 15 zeigt eine Variante von Fig. 13, bei der das Mehrdrahtinstrument aus vier Einzeldrähten 6 statt zwei Einzeldrähten gebildet ist, d.h. im distalen Funktionsabschnitt bildet sich im Funktionszustand ein Drahtkörbchen 7a aus den vier im 90°-Winkelabstand aufgebogenen Einzeldrähten 6 statt der im Beispiel von Fig. 13 aus zwei Einzeldrähten gebildeten Körbchen-/Schlingeneinheit, und der Schaftabschnitt 9a besteht aus vier verdrillten Einzeldrähten.

Fig. 16 zeigt eine weitere Variante von Fig. 3, bei der das Mehrdrahtinstrument aus sechs Einzeldrähten 6 aufgebaut ist, die im distalen Funktionsabschnitt, wenn sie sich im Funktionszustand befinden, im 60°-Winkelabstand zu einer entsprechenden Körbcheneinheit 7b aufgebogen sind und im Schaftabschnitt 9b einstückig bis zum proximalen Ende des Mehrdrahtinstruments unter Verdrillung weitergeführt sind Im distalen Endbereich sind sie unverdrillt (nicht beansprucht) oder alternativ erfindungsgemäβ ebenfalls unter Verdrillung zum distalen Abschlusselement 13a geführt und dort fixiert.

Die gezeigten und vorstehend beschriebenen Ausführungsbeispiele machen deutlich, dass die Erfindung ein Mehrdrahtinstrument bereitstellt, das sich mit relativ geringen Querschnitten realisieren lässt und das Risiko minimiert, dass an unerwünschten Stellen Drahtverbindungen unter Belastung reißen. Dazu trägt zum einen bei, dass das Mehrdrahtinstrument durchgehend aus sich einstückig vom distalen bis zum proximalen Ende des Mehrdrahtinstruments erstreckenden Einzeldrähten gebildet ist, die im Schaftabschnitt miteinander verdrillt und/oder in anderer Weise aneinander gehalten sind, z.B. durch Schweißpunkte, während sie im distalen Funktionsabschnitt im Funktionszustand ein gefordertes körbchenbildendes Funktionsdrahtgeflecht bilden, z.B. auch als eine Schlingen- oder Filtereinheit. Zum anderen sind die Einzeldrähte vor dem distalen Funktionsabschnitt an ihrem distalen Ende zusammengeführt und direkt miteinander oder an einem distalen Abschlusskörper durch eine jeweilige Sollbruchverbindung fixiert, die auf eine definierte Höchstbelastung ausgelegt ist und im Überlastfall erwünschterweise reißt, wobei sie vorzugsweise auch in ihrem distalen Endbereich zwischen dem körbchenbildenden Funktionsabschnitt und der jeweiligen Sollbruchverbindung verdrillt sind.

Das erfindungsgemäße Mehrdrahtinstrument besitzt folglich keinen weiteren Längsdrahtabschnitt, mit dem die Einzeldrähte verbunden werden müssten, und somit auch keine Drahtverbindungsstellen zwischen seinem distalen und proximalen Ende, so dass auch nicht die Gefahr besteht, dass entsprechende Verbindungsstellen unbeabsichtigt reißen. Für die Einzeldrähte sind vorzugsweise superelastische Materialien z.B. eine NiTi-Legierung wie Nitinol, aber auch für solche Anwendungszwecke geläufige Stahlmaterialien etc. verwendbar. Jeder Einzeldraht kann je nach Bedarf aus monofilem Drahtmaterial oder Litzenmaterial bestehen. Durch die Gefügeänderung im Material ist es relativ einfach, die Fixierung der distalen Einzeldrahtenden aneinander oder an einem distalen Abschlusskörper wie gewünscht als Sollbruchverbindung auszulegen. Im Vergleich zu Mehrdrahtinstrumenten, bei denen die Einzeldrähte für den distalen Funktionsabschnitt aus einem Rohrstück geschnitten sind, das im anschließenden proximalen Bereich als Schaftabschnitt fungiert, sind beim erfindungsgemäßen Mehrdrahtinstrument geringere Querschnitte bzw. Durchmesser auch im Schaftabschnitt und damit eine erhöhte Steifigkeit bei gleichem Nenndurchmesser erzielbar. So zeigt sich, dass erfindungsgemäße Mehrdrahtinstrumente, die in Steinfangkorbinstrumenten verwendbar sind, mit Schaftaußendurchmessern kleiner als 2,0 mm durch das Verdrillen der durchgehenden Einzeldrähte ohne Weiteres hergestellt werden können und die geforderten Funktionen erfüllen, wobei die Gesamtlänge des Mehrdrahtinstruments für endoskopische Anwendungen typischerweise im Bereich von etwa einem Meter bis zu mehreren Metern liegt.

Es versteht sich, dass die Erfindung nicht auf endoskopische Anwendungen beschränkt ist, sondern auf allen medizinischen und anderen Gebieten einsetzbar ist, in denen Mehrdrahtinstrumente mit einem Funktionsdrahtgeflecht aus Einzeldrähten verwendet werden.

## Patentansprüche

1. Mehrdrahtinstrument, insbesondere für Endoskope, mit
- einem distalen Funktionsabschnitt mit einem körbchenbildenden Funktionsabschnitt (5), in welchem mehrere Einzeldrähte (6) ein Drahtkörbchen bilden, wozu sie dort mit körbchenbildendem Abstand voneinander aufgebogen verlaufen und an einem distalen Ende (6a) zusammengeführt und miteinander verbunden sind, indem sie unter Bildung einer jeweiligen Sollbruchverbindung (15) direkt aneinander oder an einem distalen Abschlusskörper (13) festgelegt sind, und
- einem sich zwischen dem distalen Funktionsabschnitt und einem proximalen Endbereich (8) des Mehrdrahtinstruments erstreckenden Schaftabschnitt (9),
- wobei sich die Einzeldrähte einstückig von ihrem distalen Ende zum proximalen Endbereich (8) des Mehrdrahtinstruments erstrecken und der Schaftabschnitt (9) von den dort zusammengeführten und aneinander gehaltenen Einzeldrähten gebildet ist,
**dadurch gekennzeichnet, dass**
- die Einzeldrähte (6) im gesamten Schaftabschnitt bis zum körbchenbildenden Funktionsabschnitt (5) miteinander verdrillt sind, wobei sie vom verdrillten distalen Ende (9a) des Schaftabschnitts (9) unter radialer Aufweitung in den körbchenbildenden Funktionsabschnitt (5) übergehen, und
- die Einzeldrähte (6) in einem distal an den körbchenbildenden Funktionsabschnitt anschließenden distalen Endbereich miteinander verdrillt sind und dabei entlang dieser Verdrillung aneinander anliegen.

2. Mehrdrahtinstrument nach Anspruch 1, weiter **dadurch gekennzeichnet, dass** die Einzeldrähte im körbchenbildenden Funktionsabschnitt unverdrillt sind.

3. Mehrdrahtinstrument nach Anspruch 1 oder 2, weiter **dadurch gekennzeichnet, dass** der distale Abschlusskörper ein halbkugel- oder vollkugelförmiges Abschlusselement (13, 13a) beinhaltet.

4. Mehrdrahtinstrument nach einem der Ansprüche 1 bis 3, weiter **dadurch gekennzeichnet, dass** der distale Abschlusskörper ein Abschlusselement (13), an dem die distalen Einzeldrahtenden fixiert sind, und eine proximal an das Abschlusselement anschließende Schutzhülse (14) beinhaltet, in der die Einzeldrähte mit einem distalen Endbereich gefasst sind.

5. Mehrdrahtinstrument nach einem der Ansprüche 1 bis 4, weiter **dadurch gekennzeichnet, dass** die Verdrillung der Einzeldrähte in dem distalen Endbereich gleichartig und entweder gleichsinnig oder gegensinnig zu deren Verdrillung im Schaftabschnitt ist.

6. Mehrdrahtinstrument nach einem der Ansprüche 1 bis 5, weiter **dadurch gekennzeichnet, dass** die Einzeldrähte mit ihrem proximalen Ende (6b) direkt aneinander oder an einem proximalen Abschlusskörper (12) fixiert sind und/oder im proximalen Endbereich in einem Griffrohrelement (11) aufgenommen sind.

7. Mehrdrahtinstrument nach einem der Ansprüche 1 bis 6, weiter **dadurch gekennzeichnet, dass** der jeweilige Einzeldraht aus monofilem Drahtmaterial oder Litzendrahtmaterial besteht.

## Claims

1. A multi-wire instrument, particularly for endoscopes, having
- a distal functional section including a basket-forming functional section (5) in which several individual wires (6) form a small wire basket, where the wires extend in a basket-forming distance to each other in a bent-open condition and are guided together at a distal end (6) and connected with one another, in that they are fixed directly to one another or to a distal terminating body (13), while forming a respective predetermined breaking connection (15), and
- a shaft section (9) extending between the distal functional section and a proximal end region (8) of the multi-wire instrument,
- the individual wires extending in one piece from their distal end to the proximal end region (8) of the multi-wire instrument, and the shaft section (9) being formed by the individual wires combined there and held together,
**characterized in that**
- the individual wires (6) are mutually twisted in the entire shaft section to the basket-forming functional section (5), changing from the twisted distal end (9a) of the shaft section (9) while radially expanding into the basket-forming functional section (5), and
- the individual wires (6) are twisted in a distal end region distally adjoining the basket-forming functional section and rest against one another along said twisting.

2. The multi-wire instrument according to claim 1, further **characterized in that** the individual wires are untwisted in the basket-forming functional section.

3. The multi-wire instrument according to claim 1 or 2, further **characterized in that** the distal terminating body contains a hemispherical or spherical terminating element (13, 13a).

4. The multi-wire instrument according to any of claims 1 to 3, further **characterized in that** the distal terminating body contains a terminating element (13), to which the distal individual wire ends are fixed, and a protective sleeve (14) proximally adjoining the terminating element, in which protective sleeve (14) the individual wires are held with a distal end region.

5. The multi-wire instrument according to any of claims 1 to 4, further **characterized in that** the twisting of the individual wires in the distal end region takes place in the same manner and is either in the same direction or in the opposite direction to their twisting in the shaft section.

6. The multi-wire instrument according to any of claims 1 to 5, further **characterized in that** the individual wires are fixed with their proximal end (6b) directly to one another or to a proximal terminating body (12), and/or in the proximal end region, are received in a grip tube element (11).

7. The multi-wire instrument according to any of claims 1 to 6, further **characterized in that** the respective individual wire consists of a monofil wire material or of a stranded wire material.

## Revendications

1. Instrument à plusieurs fils, en particulier pour endoscopes, avec:
- une partie fonctionnelle distale avec une partie fonctionnelle (5) formant une corbeille, dans laquelle plusieurs fils individuels (6) forment une corbeille en fils, pour laquelle ils s'étendent sous forme cintrée à distance de formation de corbeille l'un de l'autre et ils sont réunis et assemblés les uns aux autres à une extrémité distale (6a), du fait qu'ils sont fixés directement les uns aux autres ou à un corps d'extrémité distal (13) en formant une jonction de rupture respective (15), et
- une partie de tige (9) s'étendant entre l'extrémité fonctionnelle distale et une région d'extrémité proximale (8) de l'instrument à plusieurs fils,
- dans lequel les fils individuels s'étendent d'une seule pièce depuis leur extrémité distale jusqu'à la région d'extrémité proximale (8) de l'instrument à plusieurs fils et la partie de tige (9) est formée par les fils individuels réunis et assemblés les uns aux autres à cet endroit,
**caractérisé en ce que**
- les fils individuels (6) sont torsadés les uns avec les autres dans toute la partie de tige jusqu'à la partie fonctionnelle formant une corbeille (5), dans lequel ils se prolongent depuis l'extrémité distale torsadée (9a) de la partie de tige (9) dans la partie fonctionnelle formant une corbeille (5) par un élargissement radial, et
- les fils individuels (6) sont torsadés les uns avec les autres dans une région d'extrémité distale se raccordant côté distal à la partie fonctionnelle formant une corbeille et s'appliquent en l'occurrence les uns sur les autres le long de cette torsade.

2. Instrument à plusieurs fils selon la revendication 1, **caractérisé en outre en ce que** les fils individuels ne sont pas torsadés dans la partie fonctionnelle formant une corbeille.

3. Instrument à plusieurs fils selon la revendication 1 ou 2, **caractérisé en outre en ce que** le corps d'extrémité distal contient un élément d'extrémité de forme hémisphérique ou entièrement sphérique (13, 13a).

4. Instrument à plusieurs fils selon l'une quelconque des revendications 1 à 3, **caractérisé en outre en ce que** le corps d'extrémité distal contient un élément d'extrémité (13), auquel les extrémités distales des fils individuels sont fixées, et une douille de protection (14) se raccordant côté proximal à l'élément d'extrémité, dans laquelle les fils individuels sont serrés sur une zone d'extrémité distale.

5. Instrument à plusieurs fils selon l'une quelconque des revendications 1 à 4, **caractérisé en outre en ce que** la torsion des fils individuels dans la zone d'extrémité distale est identique et est soit de même sens soit de sens contraire à leur torsion dans la partie de tige.

6. Instrument à plusieurs fils selon l'une quelconque des revendications 1 à 5, **caractérisé en outre en ce que** les fils individuels sont fixés par leur extrémité proximale (6b) directement les uns aux autres ou à un corps d'extrémité proximal (12) et/ou sont logés dans la région d'extrémité proximale dans un élément de manche tubulaire (11).

7. Instrument à plusieurs fils selon l'une quelconque des revendications 1 à 6, **caractérisé en outre en ce que** le fil individuel respectif se compose de matériau de fil mono-filaire ou de matériau de fil en toron.
